(19) Europäisches Patentamt / European Patent Office / Office européen des brevets

(11) **EP 3 526 709 B1**

(12) **EUROPEAN PATENT SPECIFICATION**

(45) Date of publication and mention of the grant of the patent:
**20.04.2022 Bulletin 2022/16**

(21) Application number: **16790894.6**

(22) Date of filing: **11.10.2016**

(51) International Patent Classification (IPC):
***G16B 50/50*** *(2019.01)*

(52) Cooperative Patent Classification (CPC):
**G16B 50/00; G16B 30/00; G16B 30/10; G16B 50/50**

(86) International application number:
**PCT/EP2016/074297**

(87) International publication number:
**WO 2018/068827 (19.04.2018 Gazette 2018/16)**

(54) **EFFICIENT DATA STRUCTURES FOR BIOINFORMATICS INFORMATION REPRESENTATION**

EFFIZIENTE DATENSTRUKTUREN ZUR DARSTELLUNG VON BIOINFORMATIKINFORMATIONEN

STRUCTURES DE DONNÉES EFFICACES POUR LA REPRÉSENTATION D'INFORMATIONS BIOINFORMATIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**21.08.2019 Bulletin 2019/34**

(73) Proprietor: **Genomsys SA**
**1015 Lausanne (CH)**

(72) Inventors:
• **RENZI, Daniele**
**1004 Lausanne (CH)**
• **ZOIA, Giorgio**
**1007 Lausanne (CH)**

(74) Representative: **Metroconsult Srl**
**Foro Bonaparte 51**
**20121 Milano (IT)**

(56) References cited:
**US-A1- 2015 227 686**

• **Anonymous: "CRAM format specification (version 3.0)", , 8 September 2016 (2016-09-08), XP002771305, Retrieved from the Internet: URL:https://samtools.github.io/hts-specs/CRAMv3.pdf [retrieved on 2017-06-22]**

## Description

## TECHNICAL FIELD

This invention discloses a Genomic Information Storage Layer (Genomic File Format) which defines a genomic data structure that includes the collection of heterogeneous data associated to the information generated by devices and applications related to genome sequencing, processing and analysis during the different stages of genomic data processing (the so-called "genomic information life cycle").

## BACKGROUND

Genomic or proteomic information generated by DNA, RNA, or protein sequencing machines is transformed, during the different stages of data processing, to produce heterogeneous data. In prior art solutions, these data are currently stored in computer files having different and unrelated structures. This information is therefore quite difficult to archive, transfer and elaborate.

The genomic or proteomic sequences referred to in this invention include, for example, and not as a limitation, nucleotide sequences, Deoxyribonucleic acid (DNA) sequences, Ribonucleic acid (RNA), and amino acid sequences. Although the description herein is in considerable detail with respect to genomic information in the form of a nucleotide sequence, it will be understood that the methods and systems for storage can be implemented for other genomic or proteomic sequences as well, albeit with a few variations, as will be understood by a person skilled in the art.

Document *"CRAM format specification (version 3.0)"* - hereinafter "CRAM" - published on September 8, 2016 describes the CRAM 3.0 format and its objectives, being for example full compatibility with BAM and effortless transition to CRAM from using BAM files. Said document is a reference based compression toolkit, employing GZIP. The coding structure of this document is based on GZIP as a monolithic file (see e.g. Chapter 14 of CRAM).

CRAM performs indexing by means of the creation of an external file which contains a tree-based structure for the retrieval of compressed blocks. Since CRAM suffers from the same limitations of SAM and it is silent on the notion of any data classification according to the result of the mapping process, the only indexing available in CRAM relies on the notion of genomic position. Furthermore, in formats such as BAM and CRAM where data classification is not present, it is not possible to index compressed data blocks according to the result of the mapping process apart from the generic criteria "mapped" or "unmapped". Summarizing, the compression scheme proposed in said document has a mediocre performance.

Document US 2015/0227686 A1 relates to an apparatus and a processor-implemented method, wherein the method includes aligning a reference genome with a plurality of DNA sequences. Each of the plurality of DNA sequences has a respective plurality of based. Further, the method includes classifying and sorting the plurality of read sequences based on respective numbers of mismatched bases within the plurality of read sequences to obtain a plurality of re-arranged DNA sequences.

The latest document describes the implementation of an aligner based on the Burrows Wheeler Transform. The output of the alignment process is a set of records describing how the reads map on a reference genome. The output format of such process is the textual SAM usually ordered by mapping position on a reference sequence (e.g. chromosome). The US-document is silent on any classification of reads according to the result of the mapping process and all reads are shuffled when they are sorted according to the mapping position. Therefore when an end user needs to extract only perfectly mapping reads or any other category of reads, the only way to find them is to decompress the entire file and perform post processing on the textual SAM output.

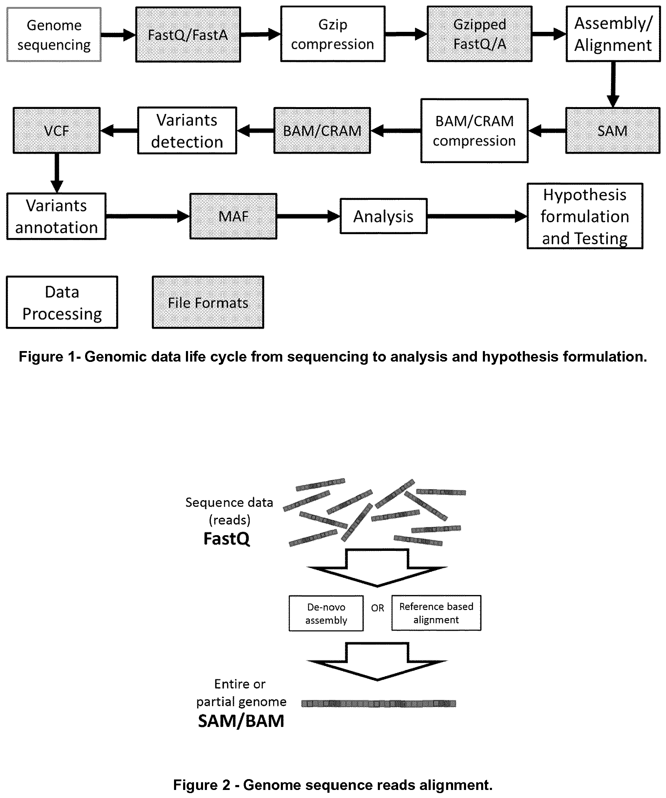

The genomic or proteomic information life cycle from data generation (sequencing) to analysis is depicted in figure 1, wherein the different phases of the genomic life cycle and the associated intermediate file formats are shown. As shown in figure 1, the typical steps of the genomic information life cycle comprise: Sequence Reads Extraction, Mapping and Alignment, Variant Detection, Variant Annotation and Functional and Structural Analysis.

Sequence Reads Extraction is the process - performed by either a human operator or a machine - of representation of fragments of genetic information in the form of sequences of symbols representing the molecules composing a biological sample. In the case of nucleic acids such molecules are called "nucleotides". The sequences of symbols produced by the extraction are commonly referred to as "reads". This information is usually encoded in prior art as "FASTA" files including a textual header and a sequence of symbols representing the sequenced molecules.

When the biological sample is sequenced to extract DNA of a living organism the alphabet is composed by the symbols (A,C,G,T,N).

When the biological sample is sequenced to extract RNA of a living organism the alphabet is composed by the symbols (A,C,G,U,N).

In case the IUPAC extended set of symbols, the so called "ambiguity codes" are also generated by the sequencing machine, the alphabet used for the symbols composing the reads is (A, C, G, T, U, W, S, M, K, R, Y, B, D, H, V, N or -).

When the IUPAC ambiguity codes are not used, a sequence of quality scores can be associated to each sequence read. In such case prior art solutions encode the resulting information as a "FASTQ" file.

Sequence alignment refers to the process of arranging sequence reads by finding regions of similarity that may be a consequence of functional, structural, or evolutionary relationships among the sequences. When the alignment is performed with reference to a pre-existing nucleotides sequence referred to as "reference sequence", the process is called "mapping". Sequence alignment can also be performed without a pre-existing sequence (i.e. reference genome) in such cases the process is known in prior art as "de novo" alignment. Prior art solutions store such information in "SAM", "BAM" or "CRAM" files. The concept of aligning sequences to reconstruct a partial or complete genome is depicted in figure 2.

Variant Detection (a.k.a. variant calling) is the process of translating the aligned output of genome sequencing machines, , to a summary of the unique characteristics of the organism being sequenced that cannot be found in other pre-existing sequences or can be found a few pre-existing sequences only. These characteristics are called "variants" because they are expressed as differences between the genome of the organism under study and a reference genome. Prior art solutions store this information in a specific file format called "VCF" file.

Variant Annotation is the process of assigning functional information to the genomic variants. This implies the classification of variants according to their relationship to coding sequences in the genome and according to their impact on the coding sequence and the gene product. This is in prior art usually stored in a "MAF" file.

Analysis of DNA strands (variant, CNV = copy number variation, methylation etc.) to define their relationship with genes (and proteins) functions and structure is called functional and structural analysis. Several different solutions exist in the prior art for the storage of this data.

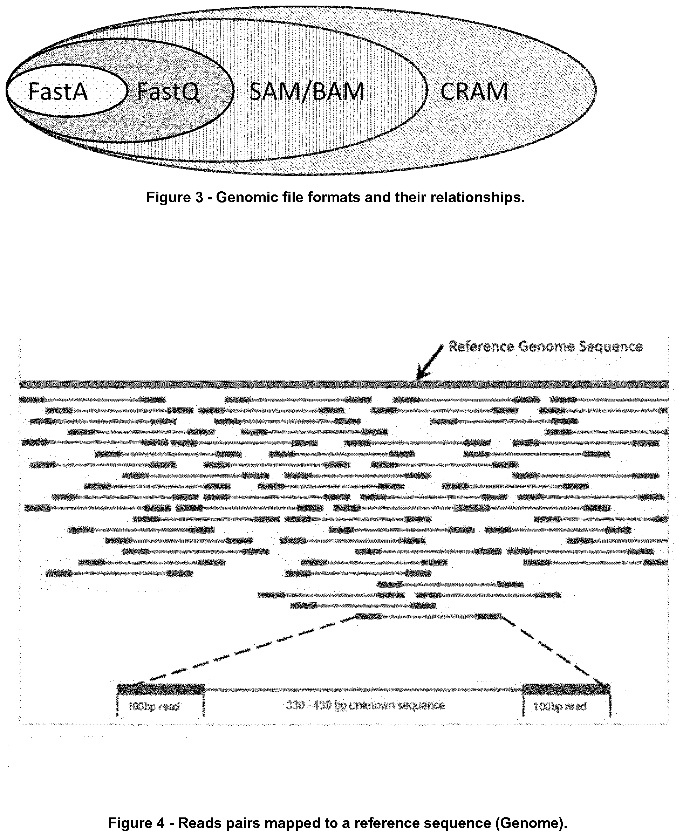

A simplified vision of the relation among the file formats used in genome processing pipelines is depicted in figure 3. In this diagram file inclusion does not imply the existence of a nested file structure, but it only represents the type and amount of information that can be encoded for each format (i.e. SAM contains all information in FASTQ, but organized in a different file structure). CRAM contains the same genomic information as SAM/BAM, but it provides more flexibility in the type of compression that can be used, therefore it is represented as a superset of SAM/BAM.

The use of multiple file formats for the storage of genomic information is highly inefficient and costly. Having different file formats at different stages of the genomic information life cycle implies a linear growth of utilized storage space even if the incremental information is very small compared to the initial volume of sequencing data. This is becoming not sustainable in terms of both space and generated costs and it is hindering genomic applications from reaching a wider portion of population. Further disadvantages of known prior art solutions are listed below.

1. Accessing, analysing or adding annotations (metadata) to raw data stored in compressed FASTQ files or any combination thereof requires decompression and recompression of the entire file with extensive usage of computational time and resources.

2. Retrieving specific type of information such as read mapping position, read variant position and type, indels position and types or any other metadata and annotation contained in aligned data stored in BAM files requires the access to the whole data volume associated to each read. Selective access to a single class of metadata is not possible with prior art solutions.

3. Prior art file formats require that the whole file is received at the end user before processing can start. For example the alignment of reads could start before the sequencing process has been completed relying on an appropriate data representation. Sequencing, alignment and analysis could proceed and run in parallel.

4. Structuring and being able of distinguishing genomic data obtained by different sequencing processes according to their specific generation semantic (e.g. sequencing obtained at different time of the life of the same individual, is not possible to be supported relying on prior art solutions. The same occurs for sequencing obtained by different types of biological samples of the same individual.

5. The encryption of entire or selected portions of the data is not supported by prior art solutions. For example the encryption of selected DNA regions; only those sequences containing variants; chimeric sequences only; unmapped sequences only; specific metadata (e.g. origin of the sequenced sample, identity of sequenced individual, type of sample) is not possible.

6. The transcoding from sequencing data aligned to a given reference (i.e. a SAM/BAM file) to a new reference requires to process the entire data volume even if the new reference differs only by a single nucleotide position from the previous reference.

7. Transfer of genomic data is slow and inefficient because the currently used data formats are organized into monolithic files of up to several hundred Gigabytes of size which need to be entirely transferred at the receiving end in order to be processed. This implies that the analysis of a small segment of the data requires the transfer of the entire file with significant costs in terms of consumed bandwidth and waiting time. Often online transfer is prohibitive for the large volumes of the data to be transferred, and the transport of the data is performed by physically moving storage media such as hard disk drives or storage servers from one location to another.

8. Processing the data is slow and inefficient for to the fact that the information is not structured in such a way that the portions of the different classes of data and metadata required by commonly used analysis applications cannot be retrieved without the need of accessing the data in its totality. This fact implies that common analysis pipelines can require to run for days or weeks wasting precious and costly processing resources for the need at each stage of accessing, parsing and filtering large volumes of data even if the portions of data relevant for the specific analysis purpose is much smaller. These limitations are preventing health care professionals from timely obtaining genomic analysis reports and promptly reacting to diseases outbreaks.

It exist a clear need to provide an appropriate genomic sequencing data and metadata representation (Genomic File Format) by organizing and partitioning the data so that the compression of data and metadata is maximized and several functionality such as selective access and support for incremental updates and other data handling functionality useful at the different stages of the genome data life cycle are efficiently enabled.

The main aspects of the disclosed solution are:

1. The classification of the sequence reads in different classes according to the results of the alignment with respect to a reference sequence in order to enable selective access to encoded data according to criteria related to the alignment results. This implies a specification of a file format that "contains" structured data elements in compressed form. Such approach can be seen as the opposite of prior art approaches, SAM and BAM for instance, in which data are structured in non-compressed form and then the entire file is compressed. A first clear advantage of the approach is to be able to efficiently and naturally provide various forms of selective access to the data elements in the compressed domain which is impossible or extremely awkward in prior art approaches.

2. The decomposition of the classified reads into layers of homogeneous metadata in order to reduce the information entropy as much as possible. The decomposition of the genomic information into specific "layers" of homogeneous data and metadata presents the considerable advantage of enabling the definition of different models of the information sources characterized by low entropy. Such models not only can differ from layer to layer, but can also differ inside each layer. This structuring enables the use of the most appropriate specific compression for each class of data or metadata and portion of them with significant gains in coding efficiency versus prior art approaches.

3. The structuring the layers into Access Units, i.e. genomic information that can be decoded either independently by using only globally available parameteres (e.g. decoder configuration) or by using information contained in other Access Units. When the compressed data within layers are partitioned into Data Blocks included into Access Units different models of the information sources characterized by low entropy can be defined.

4. The information is structured so that any relevant subset of data used by genomic analysis applications is efficiently and selectively accessible by means of appropriate interfaces. These features enable faster access to data and yield a more efficient processing. A Master Index Table and Local Index Tables enable selective access to the information carried by the layers of encoded (i.e. compressed) data without the need to decode the entire volume of compressed data. Furthermore, an association mechanism among the various data layers is specified to enable the selective access of any possible combination of subsets of semantically associated data and/or metadata layers without the need to decode all the layers.

5. The joint storage of the Master Index Table and the Access Units.

## BRIEF DESCRIPTION OF DRAWINGS

Figure 1 is a block diagram of the typical genomic information life cycle.

Figure 2 is a diagram showing the concept of aligning sequences to reconstruct a partial or complete genome is depicted.

Figure 3 is a conceptual diagram illustrating a simplified vision of the relation among the file formats used in genome processing pipelines.

Figure 4 shows reads pairs mapped to a reference sequence.

Figure 5 shows an example of Access Units according to the principles of this disclosure.

Figure 6 shows an example of Access including a header and layers composed by data blocks.

Figure 7 shows the relation among genomic "Data Packets", "Blocks", Access Units, layers and Streams Reads Classes.

Figure 8 shows a master index table with the vectors of mapping loci of the first read contained by each Access Unit.

Figure 9 shows the generic structure of the Main Header and a partial representation of MIT showing the mapping positions of the first read in each pos AU of class P.

Figure 10 shows second type of data store in the MIT.

Figure 11 shows the Access Units containing reads of class P mapped on reference sequence no. 2 between position 150,000 and 250,000 are accessed using the values contained in the T1p vector.

Figure 12 Shows a modification in the reference sequence can transform M reads in P reads.

Figure 13 is a block diagram showing the genomic information life cycle according to the principles of this invention.

Figure 14 shows a sequence reads extractor according to the principles of this invention.

Figure 15 shows a genomic encoder 2010 according to the principles of this invention.

Figure 16 shows a genomic decoder 218 according to the principles of this invention.

## SUMMARY

The aforementioned problems present in the prior art are solved by the present invention as claimed by the set of appended claims.

In one aspect, it is provided

A computer-implemented method for the storage of a representation of genome sequence data in a genomic file format, said genome sequence data comprising reads of sequences of nucleotides, comprising the steps of: aligning said reads to one or more reference sequences, thereby creating aligned reads,
classifying said aligned reads into classes comprising:

A first class when said aligned reads match with said one or more reference sequences without any error;

A second class when said aligned reads match with said one or more reference sequences with a number of mismatches;

A third class when said aligned reads match with said one or more reference sequences with the presence of substitutions of symbols and the presence of insertions or deletions and soft clipped symbols;

A fourth class when said aligned reads do not match with said one or more reference sequences, thereby creating classes of aligned reads;

entropy encoding said classified aligned reads as layers, each layer comprising data blocks, each data block comprising descriptors as a vector of syntax elements of the same type, said descriptors comprising for said first class a starting position on the reference genome, a distance to the mate pair in case of paired reads, a flag signaling if the read has to be considered as a reverse complement versus the reference, additional flags describing characteristics of the read and the value of the read length in case a sequencing technology produces variable length reads, for said second class all descriptors of said first class and a mismatch position, for said third class all descriptors of said first class, a mismatch type and a mismatch position, for said fourth class all descriptors of said first class, a mismatch position, a mismatch type and optionally a soft clipped nucleotides string when present, wherein the encoding of said classified aligned reads as layers of syntax elements is adapted according to the data or metadata carried by the layer, and wherein the encoding of said classified aligned reads as layers of syntax data elements is further adapted according to the statistical properties of the data or metadata carried by the layer;

structuring said layers of syntax elements with header information thereby creating successive access units,

creating a master index table, containing one section for each class of aligned reads, comprising the mapping positions on said one or more reference sequences of the first read of each access units of each class of data;

jointly storing said master index table and said access unit data.

By jointly storing index tables and said representation of the genome sequence data, instead of different separate files for each type of data of the genome sequence data representation as mentioned in the above life cycle description, many advantages are immediately apparent, namely:

• The results of any intermediate stage of genome sequence data processing can be incrementally added to the existing data without the need to transcode to a different file format. For example alignment information can be added to the raw data without the need to change the existing file format. Variants calling results can be included in the existing aligned sequence data with an incremental update.

- The genomic sequence data can be retrieved according to their specific characteristics without the need to access the entire file or regions thereof that do not match the criteria of the query. For example queries can be executed to selectively access:

  o Sequence reads that perfectly match on one or more reference genomes
  o Sequence reads that contain only mismatches where an "N" symbol is present instead of an actual nucleotide or amino acid symbol
  o Sequence reads that contain any type of mismatch in the form of substitution of symbols with respect to one or more genomes
  o Sequence reads that contain mismatches and insertion or deletions (indels)
  o Sequence reads that contain mismatches, insertion or deletions (indels) and soft clipped symbols with respect to one or more reference genomes
  o Sequence reads that cannot be mapped with respect to the considered reference genome(s)
  o All the Single Nucleotide Polymorphisms (SNPs) which are present between specified thresholds of depth
  o All chimeric sequence reads
  o All sequence reads with quality scores above a specified threshold
  o All metadata associated to a specified set of sequence reads

By classifying aligned reads according to a matching confidence with the reference sequence, selective access to encoded data according to criteria related to the alignment results can be achieved.

By encoding the classified aligned reads as layers of syntax elements, the encoding may be adapted according to the specific features of the data or metadata carried by the layer and its statistical properties.

By structuring the layers of syntax elements with header information in successive access units, the encoding, storage and transmission can be adapted according to the nature of the data. For example, the encoding can be adapted per access unit to use the most efficient source model for each data layer in terms of minimization of the entropy.

In accordance with one aspect, a method to extract reads of sequences of nucleotides stored in a genomic file is disclosed, wherein said genomic file comprises a master index table and access units data stored according to the principles of this disclosure, said method comprising the steps of:

receiving user input identifying the type of reads to be extracted,

retrieving vectors of data indicating the position on the reference genome of the first read in each access unit and retrieving data indicating a physical position of access units per each class of data; and

retrieving the master index table from said genomic file,

retrieving the access units corresponding to said type of reads to be extracted,

reconstructing said reads of sequences of nucleotides mapping said retrieved access units on one or more reference sequences.

The present invention further discloses a Genome Sequencing Machine comprising:

a genome sequencing unit, configured to output reads of sequences of nucleotides from a biological sample,

an alignment unit, configured to align said reads to one or more reference sequences thereby creating aligned reads,

a classification unit, configured to classify said aligned reads into classes at least comprising:

a first class when said aligned reads match with said one or more reference sequences without any error;

a second class when said aligned reads match with said one or more reference sequences with a number of mismatches;

a third class when said aligned reads match with said one or more references sequences with the presence of substitutions of symbols and the presence of insertions or deletions and soft clipped symbols;

a fourth class when said aligned reads do not match with said one or more reference sequences;

thereby creating classes of aligned reads;

an encoding unit, configured to encode said classified aligned reads as layers of encoded data, each layer comprising data blocks, each data block comprising descriptors as a vector of data elements of the same type, said descriptors used to identify said reads classified according to the mapping on the reference sequence, said descriptors comprising information data related to the position of the read with respect to the reference sequence, wherein the encoding of said classified aligned reads as layers of syntax elements is adapted according to the specific features of the data or metadata carried by the layer,

a subdividing unit, configured to structure said layers of data blocks with header information thereby creating successive access units,

an index table processing unit, configured to create a master index table, containing one section for each class of aligned reads, comprising the mapping positions on the reference sequence of the first read of each access unit of each class of data;

a storage unit, configured to jointly store said master index table and said access unit data.

In accordance with one aspect, an extractor to extract reads of sequences of nucleotides stored in a genomic file is disclosed, wherein said genomic file comprises a master index table and access units data stored according to the principles of this disclosure, said extractor comprising:

user input means configured to receive input parameters identifying the type of reads to be extracted,

retrieving means configured to retrieve said master index table from said genomic file,

retrieving means configured to retrieve the access units corresponding to said type of reads to be extracted, said retrieving means further comprising means for extracting vectors of data indicating the position on the reference genome of the first read in each access unit, said retrieving means further comprising means for retrieving data indicating a physical position of access units per each class of data,

reconstructing means configured to reconstruct said reads of sequences of nucleotides mapping said retrieved access units on one or more reference sequences.

In accordance with one disclosed aspect, a digital processing apparatus is programmed to perform a method as set forth in the immediately preceding paragraph. In accordance with another disclosed aspect, a non-transitory storage medium is accessed by a digital processing apparatus and stores instructions executable by the digital processing apparatus to perform a method as set forth in the preceding paragraph.
In accordance with another disclosed aspect, a non-transitory storage medium is readable by a digital processor and stores software for processing genomic or proteomic data represented as genomic or proteomic character strings comprising characters of a bioinformatics character set wherein each base or peptide of the genomic or proteomic data is represented in the format described in the preceding paragraphs. In some embodiments the software processes the genomic or proteomic data using digital signal processing transformations.

## DETAILED DESCRIPTION

### Classification of the sequence reads

The sequence reads generated by sequencing machines are classified by the disclosed invention into five different "classes" according to the results of the alignment with respect to one or more reference sequences.
When aligning a DNA sequence of nucleotides with respect to a reference sequence five are the possible results:

1. A region in the reference sequence is found to match the sequence read without any error (perfect mapping). Such sequence of nucleotides will be referenced to as "perfectly matching read" or denoted as "Class P".
2. A region in the reference sequence is found to match the sequence read with a number of mismatches constituted by a number of positions in which the sequencing machine was not able to call any base (or nucleotide). Such mismatches are denoted by an "N". Such sequences will be referenced to as "N mismatching reads" or "Class N".
3. A region in the reference sequence is found to match the sequence read with a number of mismatches constituted by a number of positions in which the sequencing machine was not able to call any base (or nucleotide) OR a different base than the one reported in the reference genome has been called. Such type of mismatch is called Single Nucleotide Variation (SNV) or Single Nucleotide Polymorphism (SNP). The sequence will be referenced to as "M mismatching reads" or "Class M".
4. A fourth class is constituted by sequencing reads presenting a mismatch type that includes the same mismatches of class M plus the presence of insertions or deletions (a.k.a. *indels*). Insertions are represented by a sequence of one or more nucleotides not present in the reference, but present in the read sequence. In literature when the inserted sequence is at the edges of the sequence it is referred to as "soft clipped" (i.e. the nucleotides are not matching the reference but are kept in the aligned reads contrarily to "hard clipped" nucleotides which are discarded). Deletion are "holes" (missing nucleotides) in the aligned read with respect to the reference.

Such sequences will be referenced to as "I mismatching reads" or "Class I".

5. A fifth class includes all reads that do now find any valid mapping on the reference genome according to the specified alignment constraints. Such sequences are said to be Unmapped and belonging to "Class U".

Unmapped reads can be assembled into a single sequence using de-novo assembly algorithms. Once the new sequence has been created unmapped reads can be further mapped with respect to it and be classified in one of the four classes P, N, M and I.

**Decomposition of the genomic information into layers.**

Once the classification of reads is completed with the definition of the classes, further processing consists in defining a set of distinct syntax elements which represent the remaining information enabling the reconstruction of the DNA read sequence when represented as being mapped on a given reference sequence. A DNA segment referred to a given reference sequence can be fully expressed by:

- The starting position on the reference genome (pos).
- A flag signaling if the read has to be considered as a reverse complement versus the reference (rcomp).
- A distance to the mate pair in case of paired reads (pair).
- The value of the read length in case of the sequencing technology produces variable length reads. In case of constant reads length the read length associated to each reads can obviously be omitted and can be stored in the main file header.
- Additional flags describing specific characteristics of the read (duplicate read, first or second read in a pair etc... ).
- For each mismatch:

    o Mismatch position (*nmis* for class N, *snpp* for class M, and *indp* for class I)
    o Mismatch type (not present in class N, *snpt* in class M, *indt* in class I)

- Optional soft clipped nucleotides string when present *(indc* in class I).

This classification creates groups of descriptors (syntax elements) that can be used to univocally represent genome sequence reads. The table below summarizes the syntax elements needed for each class of aligned reads.

**Table 1- Defined layers per class of data.**

|        | P | N | M | I |
|--------|---|---|---|---|
| pos    | X | X | X | X |
| pair   | X | X | X | X |
| rcomp  | X | X | X | X |
| flags  | X | X | X | X |
| rlen   | X | X | X | X |
| nmis   |   | X |   |   |
| snpp   |   |   | X |   |
| snpt   |   |   | X |   |
| indp   |   |   |   | X |
| indt   |   |   |   | X |
| indc   |   |   |   | X |

Reads belonging to class P are characterized and can be perfectly reconstructed by only a position, a reverse complement information and a distance between mates in case they have been obtained by a sequencing technology yielding mated pairs, some flags and a read length.

Figure 4 illustrates how reads can be coupled in pairs (according to the most common sequencing technology from Illumina Inc.) and mapped onto a reference sequence. Reads pairs mapped on the reference sequence are encoded into a multiplicity of layers of homogeneous descriptors (i.e. positions, distances between reads in one pair, mismatches etc....).

A layer is defined as a vector of descriptors related to one of the multiplicity of elements needed to uniquely identify the reads mapped on the reference sequence. The following are examples of layers carrying each of them a vector of descriptors:

- Reads position layer
- Reverse complement layer
- Pairing information layer
- Mismatch position layer
- Mismatch type layer
- Indels layer
- Clipped bases layer
- Reads lengths layer (present only in case of variable reads length)
- BAM flags layer

**Data Blocks, Access units and Genomic Data Layer**

The data structure further disclosed by this invention relies on the concepts of:

    **A Data Block** is defined as a set of the descriptor vector elements, of the same type (e.g. positions, distances, reverse complement flags, position and type of mismatch) composing a layer. One layer is typically composed by a multiplicity of data blocks. A data block can be partitioned into Genomic Data Packets which consist in transmission units having a size typically specified according to the communication channel requirements. Such partitioning feature is desirable for achieving transport efficiency using typical network communication protocols.

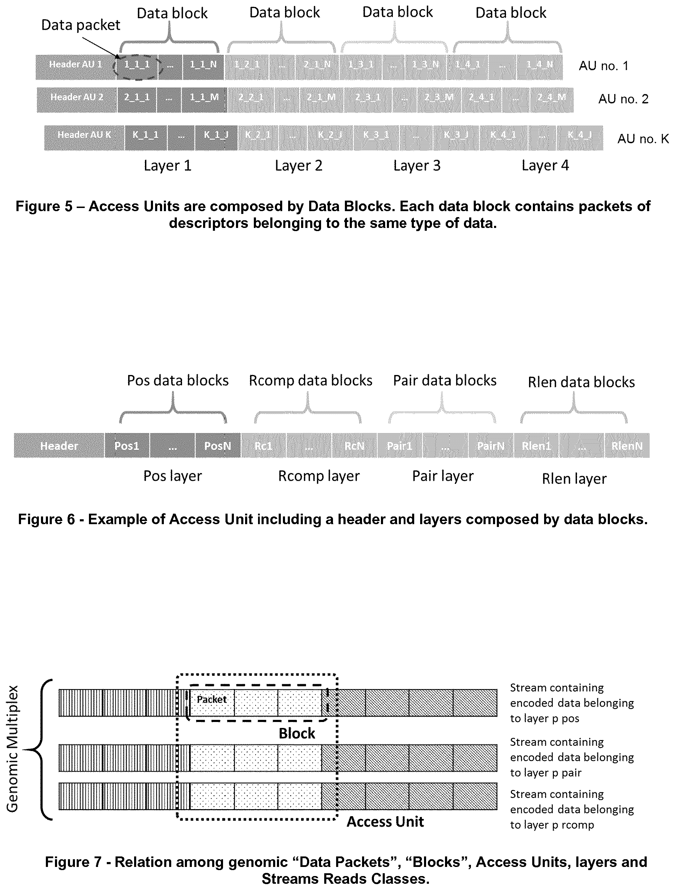

**An access unit** is defined as a subset of genomic data that can be fully decoded either independently from other access units by using only globally available data (e.g. decoder configuration) or by using information contained in other access units. An access unit is composed by a header and by the result of multiplexing data blocks of different layers. Several packets of the same type are encapsulated in a block and several blocks are multiplexed in one access unit. These concepts are depicted in Figure 5. Figure 6 shows an access unit consisting of a header and one or more layers of data blocks of the same nature. Figure 6 shows an example of a generic access unit structure depicted in Figure 5 in which

- data blocks of layer 1 contain information related to the position of reads on a reference sequence;
- data blocks of layer 2 contain information about reverse complementarity of reads;
- data blocks of layer 3 contain information related to read pairing information;
- data blocks of layer 4 contain information about the reads length.

A **Genomic Data Layer** is defined as a set of genomic data blocks encoding data of the same type (e.g. position blocks of reads perfectly matching on a reference genome are encoded in the same layer).

A **Genomic Data Stream** is a packetized version of a Genomic Data Layer where the encoded genomic data is carried as payload of Genomic Data Packets including additional service data in a header. See Figure 7 for an example of packetization of 3 Genomic Data Layers into 3 Genomic Data Stream.

A **Genomic Data Multiplex** is defined as a sequence of Genomic Access Units used to convey genomic data related to one or more processes of genomic sequencing, analysis or processing. Figure 7 provides a schematic of the relation among a Genomic Multiplex carrying three Genomic Data Streams decomposed in Access Units. The Access Units encapsulate Data Blocks belonging to the three streams and partitioned into Genomic Packets to be sent on a transmission network.

**Source models, entropy coders and coding modes.**

For each layer of the genomic data structure disclosed in this invention different coding algorithms may be employed according to the specific features of the data or metadata carried by the layer and its statistical properties. The "coding algorithm" has to be intended as the association of a specific "source model" of the descriptor with a specific "entropy coder". The specific "source model" can be specified and selected to obtain the most efficient coding of the data in terms of minimization of the source entropy. The selection of the entropy coder can be driven by coding efficiency considerations and/or probability distribution features and associated implementation issues. Each selection of a specific coding algorithm will be referred to as "coding mode" applied to an entire "layer" or to all "data blocks" contained into an access unit. Each "source model" associated to a coding mode is characterized by:

- The definition of the syntax elements emitted by each source (e.g. reads position, reads pairing information, mismatches with respect to a reference sequence etc.)
- The definition of the associated probability model.
- The definition of the associated entropy coder.

For each data layer the source model adopted in one access unit is independent from the source model used by other access units for the same data layer. This enables each access unit to use the most efficient source model for each data layer in terms of minimization of the entropy.

**TABLES**

**Master Index Table**

In order to support selective access to specific regions of the aligned data, the data structure described in this document implements an indexing tool called Master Index Table (MIT). This is a multi-dimensional array containing two classes of data:

1. the loci at which specific reads map on the used reference sequences. These values contained in the MIT are the mapping positions of the first read in each pos access unit so that non-sequential access to each access unit is supported. These sections of the MIT contain one section per each class of data (P, N, M and I) and per each reference sequence.

2. pointers to the access units containing the data necessary to reconstruct the blocks of reads following those whose mapping positions are stored in the position vectors mentioned in point 1. Each vector of pointers is referred to as Local Index Table.

## Access Units mapping positions

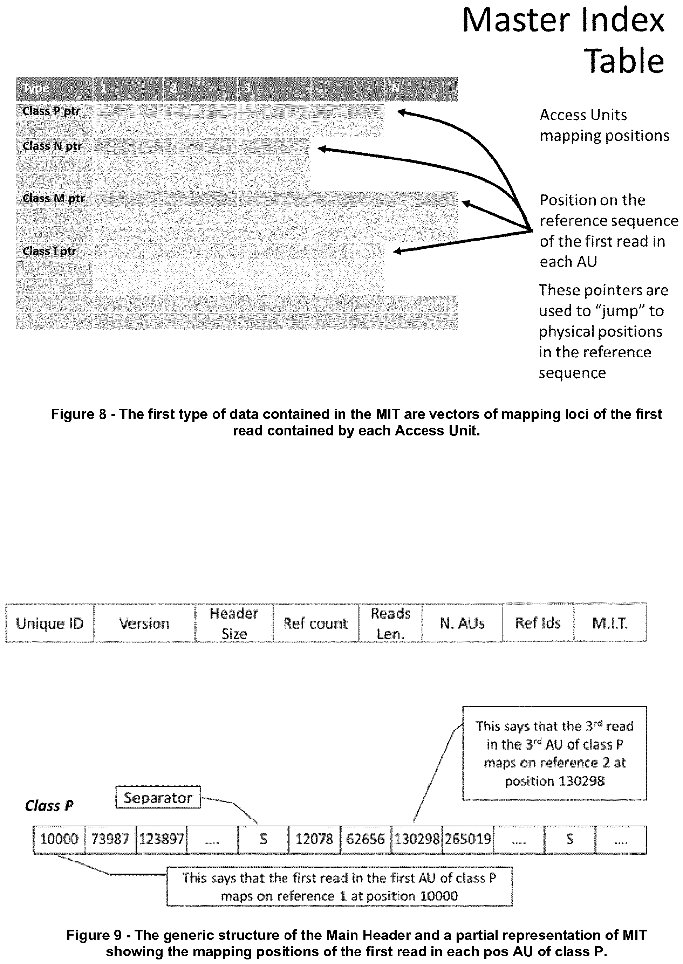

Figure 8 shows a schematic of the MIT highlighting the four vectors containing the mapping positions on the reference sequence (possibly more than one) of each access units of each class of data.

The MIT is contained in the Main Header of the encoded data. Figure 9 shows the generic structure of the Main Header and an example of MIT vector for the class P of encoded reads.

The values contained in the MIT depicted in Figure 9 are used to directly access the region of interest (and the corresponding access unit) in the compressed domain.

For example, with reference to Figure 9, if an analyst required to access perfectly matching reads mapped in the region comprised between position 150,000 and 250,000 on reference no. 2, a decoding application would skip to the class P position vector and the second reference in the MIT and would look for the two values k1 and k2 so that k1 < 150,000 and k2 > 250,000. In the example of Figure 9 this would result in positions 3 and 4 of the second block (second reference) of the MIT vector referring to mapping position of class P. These returned values will then be used by the decoding application to fetch the positions of the appropriate access units from the pos layer as described in the next section.

## Access Units Pointers

The second type of data contained in the remaining vectors of the MIT (figure 8) consists in vectors of pointers to the physical position of each access unit in the encoded bitstream. Each vector is referred to as Local Index Table since its scope is limited to one homogeneous class of encoded information.

For each of the four classes of mapped reads (P, N, M, I) several types of access units are needed to reconstruct the encoded reads (pairs). The specific types of access units associated to each class of data depends on the result of the matching function applied on the reads in each classes with respect to one or more reference sequences as described above.

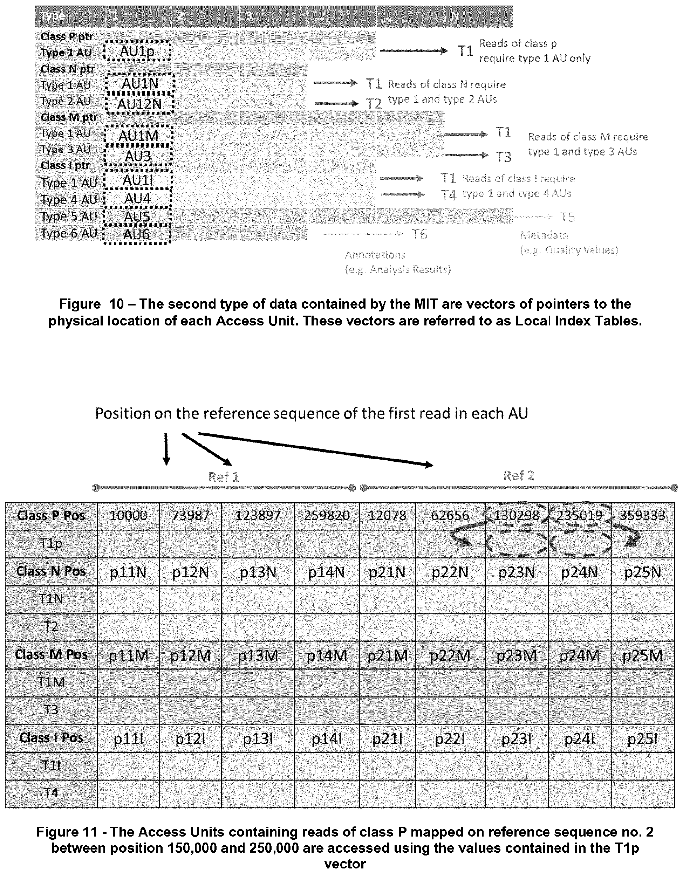

In the previous example of figure 9, in order to access region 150,000 to 250,000 of reads aligned on the reference sequence no. 2, the decoding application retrieved positions 3 and 4 from the positions vector of class P in the MIT. These values shall be used by the decoding process to access the 3rd and 4th elements of the corresponding access units vector (in this case the second) of the MIT. In the example shown in Figure 11, the Total Access Units counters contained in the Main Header are used to skip the positions of access units related to reference 1 (4 in the example). The indexes containing the physical positions of the requested access units in the encoded stream are therefore calculated as:

Position of requested AU = AUs of reference 1 to be skipped + position retrieved using the MIT i.e.

First AU position: 4 + 3 = 7

Last AU position: 4 + 4 = 8

This means that the region of interest (class P reads mapped on reference sequence no. 2 between position 150,000 and 250,000 is contained in the access units pointed to by the pointers stored in the 7th and 8th column of the Master Index Table, row T1p (Type 1 Access Units of type p).

Figure 11 shows how elements of one vector of the MIT (e.g. Class P Pos) point to elements of one LIT (Type 1 pos vector in the example of figure 11).

## Adapting the reference sequence

The mismatches encoded for classes N, M and I can be used to create a "modified genome" to be used to re-encode reads in the N, M or I layer (with respect to the first reference genome, $R_0$) as p reads with respect to the "adapted" genome $R_1$. For example if denoted with $r_{in}^M$ the $i^{th}$ read of class M containing mismatches with respect to the reference genome $n$, then after "adaptation" it couldbe obtained $r_{i(n+1)}^P$ with $A(Ref_n)=Ref_{n+1}$ where A is the transformation from reference sequence $n$ to reference sequence $n + 1$.

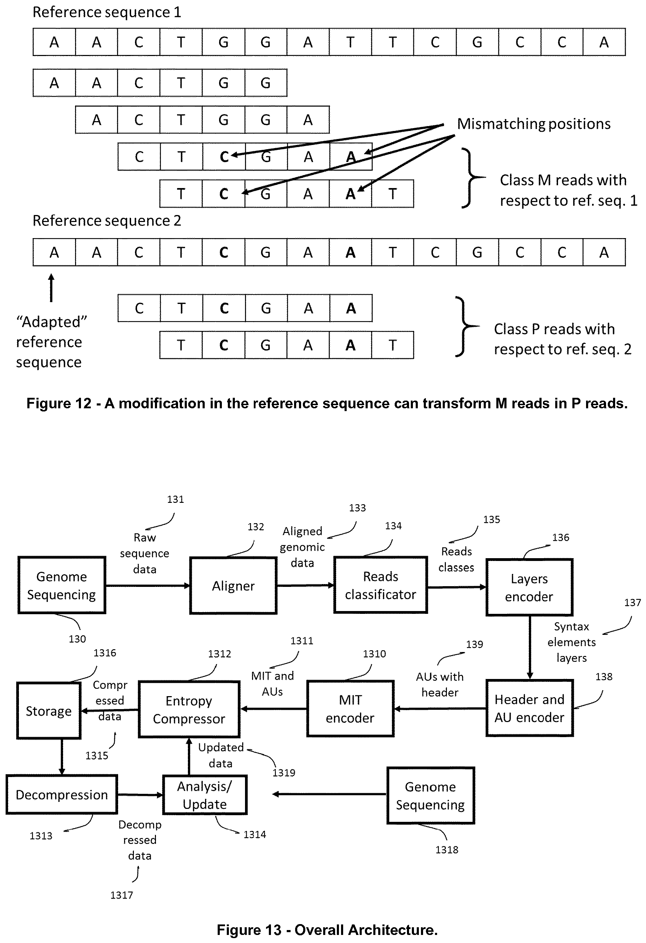

Figure 12 shows how reads containing mismatches (M reads) with respect to reference sequence 1 (RS1) can be transformed into perfectly matching reads (P reads) with respect to reference sequence 2 (RS2) obtained from RS1 by modifying the mismatching positions. This transformation can be expressed as

$$RS2 = A(RS1)$$

If the expression of transformation A which goes from RS1 to RS2 requires less bits of the expression of the mismatches present in the M reads, this encoding method results in a smaller information entropy and therefore better compression.

In some circumstances one or more modifications in the reference genome can reduce the overall information entropy by transforming a set of N, M or I reads to P reads.

A system architecture according to the principles of this invention is now described according to Figure 13. At a source, one or more genome sequencing devices 130 and/or applications generate and represent genomic information 131 in a format which contains

- One or more sequences of symbols representing nucleic acids
- A unique identifier per each genomic sequence
- An optional quality value per each symbol

- Optional metadata
- One or more optional reference sequences to be used to further process the generated genomic sequences.

A reads alignment unit 132 receives the raw sequence data and either aligns them on one or more available reference sequences or assembles them in longer sequences by looking for overlapping prefixes and suffixes applying a method known as "de-novo" assembly.

A reads classification unit 134 receives the aligned genome sequence data 133 and applies a matching function to each sequence with respect to:

- one or more available reference sequences or
- to an internal reference built during the alignment process (in case of "de-novo" assembly) .

A layers encoding unit 136 receives the reads classes 135 produced by the classification unit 134 and produces layers of syntax elements 137.

A header and Access Units encoding unit 138 encapsulates the syntax elements layers 137 in Access Units and adds a header to each Access Unit.

A Master Index Table encoding unit 1310 creates an index of pointers to the received Access Units 139

A compression unit 1312 transforms the output of said representation in a more compact (compressed) format 1315 to reduce the utilized storage space;

A local or remote storage device 1316 stores the compressed information 1315.

A decompression unit 1313 decompresses compressed information 1315 to retrieve decompressed data 1317 equivalent to genomic information 131.

An analysis unit 1314 further processes said genomic information 1317 by incrementally updating the metadata contained therein.

One or more genome sequencing devices or applications 1318 might add additional information to the existing genomic data by adding the results of a further genomic sequencing process without the need to re-encode the existing genomic information; to produce updated data 1319. Alignment and compression shall be applied to the newly generated genomic data prior to merging them with the existing data.

One of the several advantages of the embodiment described above is that genome analysis devices and application which need to have access to the data will be able to query and retrieve the needed information by using one or more of the index tables.

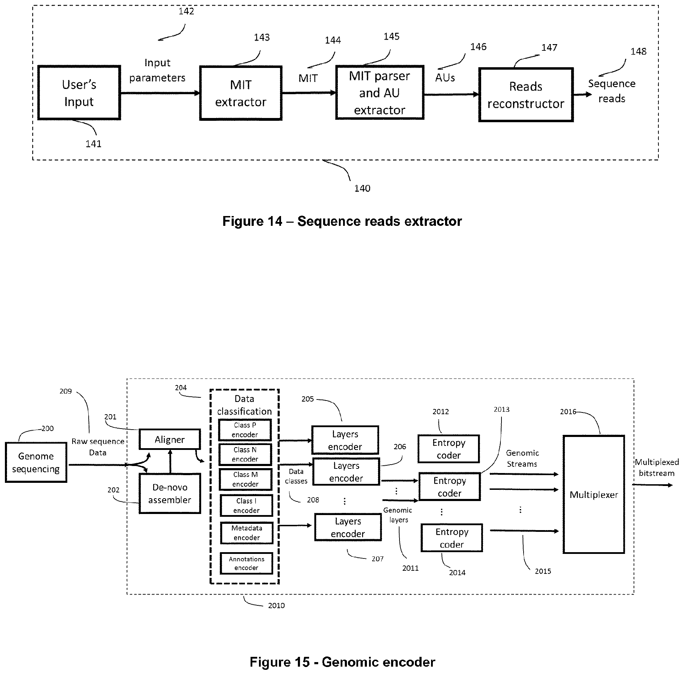

A sequence reads extractor 140 according to the principles of this invention is disclosed in figure 14.

The extractor device 140 utilises the Master Index Table described in this invention to have random access to any sequence reads stored in a Genomic File Format according to this disclosure. The extractor device 140 comprises user input means 141 to receive from the user input information 142 on the specific data to be retrieved. For example the user can specify:

a. A genomic region in terms of:

i. Start and end absolute position on a reference genome
ii. One entire reference sequence (e.g. a chromosome)

b. Only one specific type of encoded sequence reads such as:

i. Sequence reads that perfectly match on one or more reference sequences
ii. Sequence reads which present exactly N mismatches with respect to one or more reference sequences
iii. Sequence reads which present a number of mismatches with respect to one or more reference sequences below or above a specified threshold
iv. Sequence reads which present inserts and deletions with respect to a reference sequence.

The MIT extractor 143 of figure 14 parses the main header of the Genomic File to access the contained information as depicted in Figure 9:

c. A unique identifier
d. The version of the used syntax
e. The size in byte of the main header
f. The number of reference sequences used to encode the sequence reads
g. The number of data blocks contained by the stream
h. The references identifiers
i. The Master Index Table.

The MIT parser and AU extractor 145 retrieves the requested access units by exploiting the following information of the Master Index Table:

j. vectors of the positions on the reference genome of the first read in each access unit. Figure 9 shows how the decoding device can read such position and find which Access Unit contain the encoded reads mapped within the requested region.
k. The Local Index Table of each encoded layer. These vectors are used to retrieve the physical position of those access units identified in steps a which contain the sequence reads mapped on the genomic region requested by a user
l. The Local Index Tables are defined per each class of data, therefore the extractor device will extract only those classes referring to the sequence reads requested by the user. For example in case of a request for perfectly matching reads only, the extracted device will access only the LIT related to class P as depicted in Figure 8.

Using the information found in the retrieved access units and the one or more reference sequences either encoded in the genomic bitstream or available at the extracting device, the reads reconstructor 147 is able to reconstruct the original sequence reads.

Figure 15 shows an encoding apparatus 207 according to the principles of this invention. Encoding apparatus further clarifies the compression aspects of the system architecture of figure 13, however the Master Index Table and access units creation are omitted in the encoder of figure 15, which produces a compressed stream without those metadata and structuring information. The encoding apparatus 207 receives as input a raw sequence data 209, for example produced by a genome sequencing apparatus 200. Genome sequencing apparatus 200 are known in the art, like the Illumina HiSeq 2500 or the Thermo-Fisher Ion Torrent devices. The raw sequence data 209 is fed to an aligner unit 201, which prepares the sequences for encoding by aligning the reads to a reference sequence. Alternatively, a de-novo assembler 202 can be used to create a reference sequence from the available reads by looking for overlapping prefixes or suffixes so that longer segments (called "contigs") can be assembled from the reads. After having been processed by a de-novo assembler 202, reads can be mapped on the obtained longer sequence. The aligned sequences are then classified by data classification module 204. The data classes 208 are then fed to layers encoders 205-207. The genomic layers 2011 are then fed to arithmetic encoders 2012-2014 which encode the layers according to the statistical properties of the data or metadata carried by the layer. The result is a genomic stream 2015.

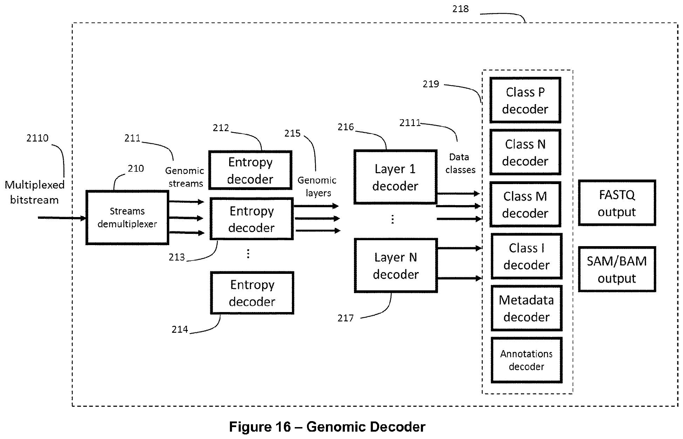

Figure 16 shows a corresponding decoding apparatus 218. A decoding apparatus 218 receives a multiplexed genomic bitstream 2110 from a network or a storage element. The multiplexed genomic bitstream 2110 is fed to a demultiplexer 210, to produce separate streams 211 which are then fed to entropy decoders 212-214, to produce genomic layers 215. The extracted genomic layers are fed to layer decoders 216-217 to further decode the layers into classes of data. Class decoders 219 further process the genomic descriptors and merge the results to produce uncompressed reads of sequences, which can then be further stored in the formats known in the art, for instance a text file or zip compressed file, or FASTQ or SAM/BAM files. Class decoders 219 are able to reconstruct the original genomic sequences by leveraging the information on the original reference sequences carried by one or more genomic streams. In case the reference sequences are not transported by the genomic streams they must be available at the decoding side and accessible by the class decoders.

In one or more examples, the inventive techniques herewith disclosed may be implemented in hardware, software, firmware or any combination. When implemented in software, these may be stored on a computer medium and executed by a hardware processing unit. The hardware processing unit may comprise one or more processors, digital signal processors, general purpose microprocessors, application specific integrated circuits or other discrete logic circuitry.

The techniques of this disclosure may be implemented in a variety of devices or apparatuses, including mobile phones, desktop computers, servers, tablets and the like.

**Claims**

1.  A computer-implemented method for the storage of a representation of genome sequence data in a genomic file format, said genome sequence data comprising reads of sequences of nucleotides, comprising the steps of:

    - aligning said reads to one or more reference sequences, thereby creating aligned reads;
    - classifying said aligned reads into classes at least comprising:

        - a first class when said aligned reads match with said one or more reference sequences without any error;
        - a second class when said aligned reads match with said one or more reference sequences with a number of mismatches;
        - a third class when said aligned reads match with said one or more reference sequences with the presence of substitutions of symbols and the presence of insertions or deletions and soft clipped symbols;
        - a fourth class when said aligned reads do not match with said one or more reference sequences,

        thereby creating classes of aligned reads;

    - entropy encoding said classified aligned reads as layers, each layer comprising data blocks, each data block comprising descriptors as a vector of syntax data elements of the same type, said descriptors comprising for said first class a starting position (pos) on the reference genome, a distance to the mate pair in case of paired reads (pair), a flag signaling if the read has to be considered as a reverse complement versus the reference (rcomp), additional flags describing characteristics of the read and the value of the read length (rlen) in case a sequencing technology produces variable length reads, for said second class all descriptors of said first class and a mismatch position (nmis), for said third class all descriptors of said first class, a mismatch type (snpt) and a mismatch position (snpp), for said fourth class all descriptors of said first class, a mismatch position (indp), a mismatch type (indt) and optionally a soft clipped nucleotides string when present (indc), wherein the encoding of said classified aligned reads as layers of syntax elements is adapted according to the data or metadata carried by the layer, and wherein the encoding of said classified aligned reads as layers of syntax data elements is further adapted according to the statistical properties of the data or metadata carried by the layer;
- structuring said layers of data blocks with header information thereby creating successive access units;
- creating a master index table, containing one section for each class of aligned reads, comprising the mapping positions on said one or more reference sequences of the first read of each access units of each class of aligned reads;
- jointly storing said master index table and said access unit data.

2. The method of claim 1, wherein said master index table further comprises a vector of pointers to the physical position of each subsequent access unit in an encoded bitstream.

3. The method of claim 1, wherein said master index table further contains one section for each reference sequence.

4. The method of claim 1, wherein the encoding of said classified aligned reads as layers employs different entropy coders.

5. A method to extract reads of sequences of nucleotides stored in a genomic file, wherein said genomic file comprises a master index table and access units data stored according to the method of claim 1, said method comprising the steps of:

   - receiving user input identifying the type of reads to be extracted;
   - retrieving vectors of data indicating the position on the reference genome of the first read in each access unit and retrieving data indicating a physical position of access units per each class of data; and
   - retrieving the master index table from said genomic file,
   - retrieving the access units corresponding to said type of reads to be extracted,
   - reconstructing said reads of sequences of nucleotides mapping said retrieved access units on one or more reference sequences.

6. The method of claim 5, wherein the genomic file further comprises the one or more reference sequences.

7. The method of claim 5, wherein the one or more reference sequences are provided via an out of band mechanism.

8. A Genome Sequencing Machine comprising:

   - a genome sequencing unit (130), configured to output reads of sequences of nucleotides (131) from a biological sample;
   - an alignment unit (132), configured to align said reads to one or more reference sequences, thereby creating aligned reads (133);
   - a classification unit (134), configured to classify said aligned reads into classes at least comprising:

      - a first class when said aligned reads match with said one or more reference sequences without any error;
      - a second class when said aligned reads match with said one or more reference sequences with a number of mismatches;
      - a third class when said aligned reads match with said one or more references sequences with the presence of substitutions of symbols and the presence of insertions or deletions and soft clipped symbols;
      - a fourth class when said aligned reads do not match with said one or more reference sequences;
      thereby creating classes of aligned reads (135);

   - an encoding unit (136), configured to encode said classified aligned reads as layers of encoded data, each layer comprising data blocks, each data block comprising descriptors as a vector of data elements of the same type, said descriptors used to identify said reads classified according to the mapping on the reference sequence, said descriptors comprising information data related to the position of the read with respect to the reference sequence, wherein the encoding of said classified aligned reads as layers of syntax elements is adapted according to the specific features of the data or metadata carried by the layer and its statistical properties,
   - a subdividing unit (138), configured to structure said layers of data blocks with header information thereby creating successive access units (139);
   - an index table processing unit (1310), configured to create a master index table, containing one section for each class of aligned reads, comprising the mapping positions on the reference sequence of the first read of each access unit of each class of data;
- a storage unit (1312-1316), configured to jointly store said master index table and said access unit data (1311).

**9.** The Genome Sequencing Machine of claim 8, wherein the master index table further comprises a vector of pointers to the physical position of each subsequent access unit.

**10.** An extractor (140) to extract reads of sequences of nucleotides stored in a genomic file, wherein said genomic file comprises a master index table and access units data stored according to the method of claim 1, said extractor (140) comprising:

- user input means (141) configured to receive input parameters (142) identifying the type of reads to be extracted,
- retrieving means (143) configured to retrieve said master index table (144) from said genomic file,
- retrieving means (145) configured to retrieve the access units (146) corresponding to said type of reads to be extracted, said retrieving means further comprising means for extracting vectors of data indicating the position on the reference genome of the first read in each access unit, said retrieving means further comprising means for retrieving data indicating a physical position of access units per each class of data;
- reconstructing means (147) configured to reconstruct said reads of sequences of nucleotides (148) mapping said retrieved access units on one or more reference sequences.

**11.** A machine readable medium comprising a plurality of instructions that in response to being executed on a computing device, causes the computing device to perform the method of claims 1-7.

**Patentansprüche**

**1.** Computerimplementiertes Verfahren für die Speicherung einer Repräsentation von Genomsequenz-Daten in einem genomischen Dateiformat, wobei die Genomsequenz-Daten Reads von Sequenzen von Nukleotiden umfassen, wobei das Verfahren die Schritte umfasst:

- Alignieren der Reads mit einer oder mehreren Referenzsequenzen, wodurch alignierte Reads geschaffen werden;
- Klassifizieren der alignierten Reads in Klassen, die zumindest umfassen:
- eine erste Klasse, wenn die alignierten Reads mit der einen oder den mehreren Referenzsequenzen ohne jeglichen Fehler übereinstimmen;
- eine zweite Klasse, wenn die alignierten Reads mit der einen oder den mehreren Referenzsequenzen mit einer Anzahl von Übereinstimmungsfehlern übereinstimmen;
- eine dritte Klasse, wenn die alignierten Reads mit der einen oder den mehreren Referenzsequenzen bei Vorliegen von Substitutionen von Symbolen und bei Vorliegen von Insertionen oder Deletionen und weich abgeschnittenen Symbolen übereinstimmen;
- eine vierte Klasse, wenn die alignierten Reads nicht mit der einen oder den mehreren Referenzsequenzen übereinstimmen, wodurch Klassen von alignierten Reads geschaffen werden;
- Entropie-Kodieren der klassifizierten alignierten Reads als Schichten, wobei jede Schicht Datenblöcke umfasst, wobei jeder Datenblock Deskriptoren als einen Vektor von Syntax-Datenelementen desselben Typs umfasst, wobei die Deskriptoren umfassen: für die erste Klasse eine Startposition (pos) an dem Referenzgenom, einen Abstand zu dem übereinstimmenden Paar im Fall von gepaarten Reads (pair), ein Flag, das signalisiert, wenn das Read als ein reverses Komplement in Bezug auf die Referenz angesehen werden muss (rcomp), zusätzliche Flags, die Eigenschaften des Reads und den Wert der Read-Länge (rlen) beschreiben, in dem Fall, dass eine Sequenzierungstechnologie Reads variabler Länge produziert, für die zweite Klasse alle Deskriptoren der ersten Klasse und eine Übereinstimmungsfehler-Position (nmis), für die dritte Klasse alle Deskriptoren der ersten Klasse und einen Übereinstimmungsfehler-Typ (snpt) und eine Übereinstimmungsfehler-Position (snpp), für die vierte Klasse alle Deskriptoren der ersten Klasse und eine Übereinstimmungsfehler-Position (indp), einen Übereinstimmungsfehler-Typ (indt) und optional, sofern dieser vorliegt, einen weich abgeschnittenen Nukleotid-String (indc), wobei das Kodieren der klassifizierten alignierten Reads als Schichten von Syntaxelementen in Übereinstimmung mit den Daten oder Metadaten, die von der Schicht getragen werden, adaptiert wird, und wobei das Kodieren der klassifizierten alignierten Reads als Schichten von Syntax-Datenelementen ferner in Übereinstimmung mit den statistischen Eigenschaften der Daten oder Metadaten, die von der Schicht getragen werden, adaptiert wird;
- Strukturieren der Schichten von Datenblöcken mit Header-Informationen, wodurch aufeinander folgende Zugriffseinheiten geschaffen werden;
- Schaffen einer Master-Index-Tabelle, die eine Sektion für jede Klasse von alignierten Reads enthält, umfassend die Zuordnungspositionen an der einen oder den mehreren Referenzsequenzen des ersten Reads jeder Zugriffseinheit jeder Klasse von alignierten Reads;

- verknüpftes Speichern der Master-Index-Tabelle und der Zugriffseinheits-Daten.

2. Verfahren nach Anspruch 1, wobei die Master-Index-Tabelle ferner einen Vektor von Zeigern auf die physische Position jeder darauffolgenden Zugriffseinheit in einem kodierten Bitstrom umfasst.

3. Verfahren nach Anspruch 1, wobei die Master-Index-Tabelle ferner eine Sektion für jede Referenzsequenz enthält.

4. Verfahren nach Anspruch 1, wobei die Kodierung der klassifizierten alignierten Reads als Schichten verschiedene Entropie-Kodierer einsetzt.

5. Verfahren zum Extrahieren von Reads von Sequenzen von Nukleotiden, die in einer genomischen Datei gespeichert sind, wobei die genomische Datei eine Master-Index-Tabelle und Daten über Zugriffseinheiten umfasst, die gemäß dem Verfahren nach Anspruch 1 gespeichert wurden, wobei das Verfahren die Schritte umfasst:

- Empfangen einer Benutzereingabe, welche den Typ von Reads, die extrahiert werden sollen, identifiziert;
- Abrufen von Vektoren von Daten, welche die Position auf dem Referenzgenom des ersten Reads in jeder Zugriffseinheit angeben, und Abrufen von Daten, die eine physikalische Position von Zugriffseinheiten für jede Klasse von Daten angeben; und
- Abrufen der Master-Index-Tabelle aus der genomischen Datei,
- Abrufen der Zugriffseinheiten entsprechend dem Typ von Reads, die extrahiert werden sollen,
- Rekonstruieren der Reads von Sequenzen von Nukleotiden, die den abgerufenen Zugriffseinheiten an einer oder mehreren Referenzsequenzen zugeordnet sind.

6. Verfahren nach Anspruch 5, wobei die genomische Datei ferner die eine oder die mehreren Referenzsequenzen umfasst.

7. Verfahren nach Anspruch 5, wobei die eine oder die mehreren Referenzsequenzen durch einen Out-of-the-Band-Mechanismus vorgesehen sind.

8. Genom-Sequenzierungsmaschine, umfassend:

- ein Genom-Sequenzierungseinheit (130), die dazu ausgestaltet ist, Reads von Sequenzen von Nukleotiden (131) von einer biologischen Probe auszugeben;
- eine Alignment-Einheit (132), die dazu ausgestaltet ist, die Reads mit einer oder mehreren Referenzsequenzen zu alignieren, wodurch alignierte Reads (133) geschaffen werden;
- eine Klassifizierungseinheit (134), die dazu ausgestaltet ist, die alignierten Reads in Klassen zu klassifizieren, die zumindest umfassen:
- eine erste Klasse, wenn die alignierten Reads mit der einen oder den mehreren Referenzsequenzen ohne jeglichen Fehler übereinstimmen;
- eine zweite Klasse, wenn die alignierten Reads mit der einen oder den mehreren Referenzsequenzen mit einer Anzahl von Übereinstimmungsfehlern übereinstimmen;
- eine dritte Klasse, wenn die alignierten Reads mit der einen oder den mehreren Referenzsequenzen bei Vorliegen von Substitutionen von Symbolen und bei Vorliegen von Insertionen oder Deletionen und weich abgeschnittenen Symbolen übereinstimmen;
- eine vierte Klasse, wenn die alignierten Reads nicht mit der einen oder den mehreren Referenzsequenzen übereinstimmen;
wodurch Klassen von alignierten Reads (135) geschaffen werden;
- eine Kodiereinheit (136), die dazu ausgestaltet ist,, die klassifizierten alignierten Reads als Schichten von kodierten Daten zu kodieren, wobei jede Schicht Datenblöcke umfasst, wobei jeder Datenblock Deskriptoren als einen Vektor von Datenelementen desselben Typs umfasst, wobei die Deskriptoren verwendet werden, um die klassifizierten Reads in Übereinstimmung mit der Zuordnung an der Referenzsequenz zu identifizieren, wobei die Deskriptoren Informationsdaten in Verbindung mit der Position des Reads in Bezug auf die Referenzsequenz umfassen, wobei das Kodieren der klassifizierten alignierten Reads als Schichten von Syntax-Elementen in Übereinstimmung mit den spezifischen Merkmalen der Daten oder Metadaten, die von der Schicht getragen werden, und ihren statistischen Eigenschaften adaptiert wird,
- eine Unterteilungseinheit (138), die dazu ausgestaltet ist, die Schichten von Datenblöcken mit Header-Informationen zu strukturieren, wodurch aufeinander folgende Zugriffseinheiten (139) geschaffen werden;
- eine Index-Tabellen-Verarbeitungseinheit (1310), die dazu ausgestaltet ist, eine Master-Index-Tabelle zu schaffen, die eine Sektion für jede Klasse von alignierten Reads enthält, umfassend die Zuordnungspositionen an der Referenzsequenz des ersten Reads jeder Zugriffseinheit jeder Klasse von Daten;
- eine Speichereinheit (1312-1316), die dazu ausgestaltet ist, die Master-Index-Tabelle und die Zugriffseinheits-Daten (1311) verknüpft zu speichern.

**9.** Genom-Sequenzierungsmaschine nach Anspruch 8, wobei die Master-Index-Tabelle ferner einen Vektor von Zeigern auf die physische Position jeder darauffolgenden Zugriffseinheit umfasst.

**10.** Extraktor (140) zum Extrahieren von Reads von Sequenzen von Nukleotiden, die in einer genomischen Datei gespeichert sind, wobei die genomische Datei eine Master-Index-Tabelle und Daten über Zugriffseinheiten umfasst, die gemäß dem Verfahren nach Anspruch 1 gespeichert wurden, wobei der Extraktor (140) umfasst:

- Benutzereingabemittel (141), die dazu ausgestaltet sind, Eingangsparameter (142) zu empfangen, die den Typ von Reads, die extrahiert werden sollen, identifizieren,
- Abrufmittel (143), die dazu ausgestaltet sind, die Master-Index-Tabelle (144) aus der genomischen Datei abzurufen,
- Abrufmittel (145), die dazu ausgestaltet sind, die Zugriffseinheiten (146) entsprechend dem Typ von Reads, die extrahiert werden sollen, abzurufen, wobei die Abrufmittel ferner Mittel zum Extrahieren von Vektoren von Daten umfasst, welche die Position an dem Referenzgenom des ersten Reads jeder Zugriffseinheit angeben, wobei die Abrufmittel ferner Mittel zum Abrufen von Daten umfassen, die eine physische Position von Zugriffseinheiten für jede Klasse von Daten angeben;
- Rekonstruktionsmittel (147), die dazu ausgestaltet sind, die Reads von Sequenzen von Nukleotiden (148), die den abgerufenen Zugriffseinheiten an einer oder mehreren Referenzsequenzen zugeordnet sind, zu rekonstruieren.

**11.** Maschinenlesbares Medium, umfassend eine Vielzahl von Anweisungen, die in Ansprechen darauf, dass sie auf einer Computervorrichtung ausgeführt werden, die Computervorrichtung veranlassen, das Verfahren nach den Ansprüchen 1-7 durchzuführen.

## Revendications

**1.** Procédé mis en œuvre par ordinateur pour le stockage d'une représentation de données de séquence de génome dans un format de fichier génomique, lesdites données de séquence de génome comprenant des lectures de séquences de nucléotides, le procédé comprenant les étapes ci-dessous consistant à :

- aligner lesdites lectures sur une ou plusieurs séquences de référence, ce qui permet de créer par conséquent des lectures alignées ;
- classer lesdites lectures alignées en des classes comprenant au moins :

- une première classe lorsque lesdites lectures alignées correspondent à ladite une ou auxdites plusieurs séquences de référence sans aucune erreur ;
- une deuxième classe lorsque lesdites lectures alignées correspondent à ladite une ou auxdites plusieurs séquences de référence avec un nombre de nonconcordances ;
- une troisième classe lorsque lesdites lectures alignées correspondent à ladite une ou auxdites plusieurs séquences de référence avec la présence de substitutions de symboles et la présence d'insertions ou de suppressions et de symboles à écrêtage doux ;
- une quatrième classe lorsque lesdites lectures alignées ne correspondent pas à ladite une ou auxdites plusieurs séquences de référence, ce qui permet de créer par conséquent des classes de lectures alignées ;

- mettre en œuvre un codage entropique desdites lectures alignées classées en tant que des couches, chaque couche comprenant des blocs de données, chaque bloc de données comprenant des descripteurs sous la forme d'un vecteur d'éléments de données syntaxiques du même type, lesdits descripteurs comprenant, pour ladite première classe, une position de départ (« pos ») sur le génome de référence, une distance jusqu'à la paire conjuguée dans le cas de lectures appariées (« pair »), un drapeau signalant si la lecture doit être considérée comme un complément inverse par rapport à la référence (« rcomp »), des drapeaux supplémentaires décrivant des caractéristiques de la lecture et la valeur de la longueur de lecture (« rien ») dans le cas où une technologie de séquençage produit des lectures de longueur variable ; pour ladite deuxième classe, tous les descripteurs de ladite première classe et une position de nonconcordance (« nmis ») ; pour ladite troisième classe, tous les descripteurs de ladite première classe, un type de non-concordance (« snpt ») et une position de non-concordance (« snpp ») ; pour ladite quatrième classe, tous les descripteurs de ladite première classe, une position de non-concordance (« indp »), un type de non-concordance (« indt ») et, facultativement, une chaîne de nucléotides à écrêtage doux si présente (« indc »), dans lequel le codage desdites lectures alignées classées en tant que des couches d'éléments de données syntaxiques est adapté selon les données ou métadonnées transportées par la couche, et dans lequel le codage desdites lectures alignées classées en tant que des couches d'éléments de données syntaxiques est en outre adapté selon les propriétés statistiques des données ou métadonnées transportées par la couche ;

- structurer lesdites couches de blocs de données avec des informations d'en-tête, ce qui permet de créer par conséquent des unités d'accès successives ;

- créer une table d'index maîtresse, contenant une section pour chaque classe de lectures alignées, comprenant les positions de mise en correspondance sur ladite une ou lesdites plusieurs séquences de référence de la première lecture de chaque unité d'accès de chaque classe de lectures alignées ;

- stocker conjointement ladite table d'index maîtresse et lesdites données d'unités d'accès.

2. Procédé selon la revendication 1, dans lequel ladite table d'index maîtresse comprend en outre un vecteur de pointeurs vers la position physique de chaque unité d'accès subséquente dans un train de bits codé.

3. Procédé selon la revendication 1, dans lequel ladite table d'index maîtresse contient en outre une section pour chaque séquence de référence.

4. Procédé selon la revendication 1, dans lequel le codage desdites lectures alignées classées en tant que des couches emploie différents codeurs entropiques.

5. Procédé d'extraction de lectures de séquences de nucléotides stockées dans un fichier génomique, dans lequel ledit fichier génomique comprend une table d'index maîtresse et des données d'unités d'accès stockées conformément au procédé selon la revendication 1, ledit procédé comprenant les étapes ci-dessous consistant à :

- recevoir une entrée utilisateur identifiant le type de lectures à extraire ;
- récupérer des vecteurs de données indiquant la position, sur le génome de référence, de la première lecture, dans chaque unité d'accès, et récupérer des données indiquant une position physique d'unités d'accès pour chaque classe de données ; et

- récupérer la table d'index maîtresse à partir dudit fichier génomique ;
- récupérer les unités d'accès correspondant audit type de lectures à extraire ; et
- reconstruire lesdites lectures de séquences de nucléotides en mettant en correspondance lesdites unités d'accès récupérées sur une ou plusieurs séquences de référence.

6. Procédé selon la revendication 5, dans lequel le fichier génomique comprend en outre ladite une ou lesdites plusieurs séquences de référence.

7. Procédé selon la revendication 5, dans lequel ladite une ou lesdites plusieurs séquences de référence sont fournies par l'intermédiaire d'un mécanisme hors bande.

8. Machine de séquençage de génome comprenant :

- une unité de séquençage de génome (130), configurée de manière à fournir en sortie des lectures de séquences de nucléotides (131) à partir d'un échantillon biologique ;
- une unité d'alignement (132), configurée de manière à aligner lesdites lectures sur une ou plusieurs séquences de référence, ce qui permet de créer par conséquent des lectures alignées (133) ;
- une unité de classification (134), configurée de manière à classer lesdites lectures alignées en des classes comprenant au moins :

- une première classe lorsque lesdites lectures alignées correspondent à ladite une ou auxdites plusieurs séquences de référence sans aucune erreur ;
- une deuxième classe lorsque lesdites lectures alignées correspondent à ladite une ou auxdites plusieurs séquences de référence avec un nombre de non-concordances ;
- une troisième classe lorsque lesdites lectures alignées correspondent à ladite une ou auxdites plusieurs séquences de référence avec la présence de substitutions de symboles et la présence d'insertions ou de suppressions et de symboles à écrêtage doux ;
- une quatrième classe lorsque lesdites lectures alignées ne correspondent pas à ladite une ou auxdites plusieurs séquences de référence, ce qui permet de créer par conséquent des classes de lectures alignées (135) ;

- une unité de codage (136) configurée de manière à coder lesdites lectures alignées classées en tant que des couches de données codées, chaque couche comprenant des blocs de données, chaque bloc de données comprenant des descripteurs sous la forme d'un vecteur d'éléments de données du même type, lesdits descripteurs étant utilisés pour identifier lesdites lectures classées selon la mise en correspondance sur la séquence de référence, lesdits descripteurs comprenant des données d'information connexes à la position de la lecture par rapport à la séquence de référence, dans lequel le codage desdites lectures alignées classées en tant que des couches d'éléments syntaxiques est adapté selon les caractéristiques spécifiques des données ou métadonnées transportées par la couche et selon ses propriétés statistiques ;
- une unité de subdivision (138), configurée de manière à structurer lesdites couches de blocs de données avec des informations d'en-tête, ce qui permet de créer par conséquent des unités d'accès successives (139) ;
- une unité de traitement de table d'index (1310), configurée de manière à créer une table d'index maîtresse, contenant une section pour chaque classe de lectures alignées, comprenant les positions de mise en correspondance sur la séquence de référence de la première lecture de chaque unité d'accès de chaque classe de données ; et
- une unité de stockage (1312-1316), configurée de manière à stocker conjointement ladite table d'index maîtresse et lesdites données d'unités d'accès (1311).

**9.** Machine de séquençage de génome selon la revendication 8, dans laquelle la table d'index maîtresse comprend en outre un vecteur de pointeurs vers la position physique de chaque unité d'accès subséquente.

**10.** Extracteur (140) destiné à extraire des lectures de séquences de nucléotides stockées dans un fichier génomique, dans lequel ledit fichier génomique comprend une table d'index maîtresse et des données d'unités d'accès stockées conformément au procédé selon la revendication 1, ledit extracteur (140) comprenant :

- un moyen d'entrée utilisateur (141) configuré de manière à recevoir des paramètres d'entrée (142) identifiant le type de lectures à extraire ;
- un moyen de récupération (143) configuré de manière à récupérer ladite table d'index maîtresse (144) à partir dudit fichier génomique ;
- un moyen de récupération (145) configuré de manière à récupérer les unités d'accès (146) correspondant audit type de lectures à extraire, ledit moyen de récupération comprenant en outre un moyen pour extraire des vecteurs de données indiquant la position, sur le génome de référence, de la première lecture dans chaque unité d'accès, ledit moyen de récupération comprenant en outre un moyen pour récupérer des données indiquant une position physique d'unités d'accès pour chaque classe de données ;
- un moyen de reconstruction (147) configuré de manière à reconstruire lesdites lectures de séquences de nucléotides (148) en mettant en correspondance lesdites unités d'accès récupérées sur une ou plusieurs séquences de référence.

**11.** Support lisible par machine comprenant une pluralité d'instructions qui, en réponse à leur exécution sur un dispositif informatique, amènent le dispositif informatique à mettre en œuvre le procédé selon l'une quelconque des revendications 1 à 7.

**Figure 1- Genomic data life cycle from sequencing to analysis and hypothesis formulation.**

**Figure 2 - Genome sequence reads alignment.**

**Figure 3 - Genomic file formats and their relationships.**

**Figure 4 - Reads pairs mapped to a reference sequence (Genome).**

**Figure 5 – Access Units are composed by Data Blocks. Each data block contains packets of descriptors belonging to the same type of data.**

**Figure 6 - Example of Access Unit including a header and layers composed by data blocks.**

**Figure 7 - Relation among genomic "Data Packets", "Blocks", Access Units, layers and Streams Reads Classes.**

# Master Index Table

| Type | 1 | 2 | 3 | ... | N |
|---|---|---|---|---|---|
| Class P ptr | | | | | |
| Class N ptr | | | | | |
| Class M ptr | | | | | |
| Class I ptr | | | | | |

Access Units mapping positions

Position on the reference sequence of the first read in each AU

These pointers are used to "jump" to physical positions in the reference sequence

**Figure 8 - The first type of data contained in the MIT are vectors of mapping loci of the first read contained by each Access Unit.**

| Unique ID | Version | Header Size | Ref count | Reads Len. | N. AUs | Ref Ids | M.I.T. |
|---|---|---|---|---|---|---|---|

This says that the 3rd read in the 3rd AU of class P maps on reference 2 at position 130298

*Class P*

Separator

| 10000 | 73987 | 123897 | .... | S | 12078 | 62656 | 130298 | 265019 | .... | S | .... |

This says that the first read in the first AU of class P maps on reference 1 at position 10000

**Figure 9 - The generic structure of the Main Header and a partial representation of MIT showing the mapping positions of the first read in each pos AU of class P.**

Figure 10 – The second type of data contained by the MIT are vectors of pointers to the physical location of each Access Unit. These vectors are referred to as Local Index Tables.

Position on the reference sequence of the first read in each AU

| Class P Pos | 10000 | 73987 | 123897 | 259820 | 12078 | 62656 | 130298 | 235019 | 359333 |
|---|---|---|---|---|---|---|---|---|---|
| T1p | | | | | | | | | |
| Class N Pos | p11N | p12N | p13N | p14N | p21N | p22N | p23N | p24N | p25N |
| T1N | | | | | | | | | |
| T2 | | | | | | | | | |
| Class M Pos | p11M | p12M | p13M | p14M | p21M | p22M | p23M | p24M | p25M |
| T1M | | | | | | | | | |
| T3 | | | | | | | | | |
| Class I Pos | p11I | p12I | p13I | p14I | p21I | p22I | p23I | p24I | p25I |
| T1I | | | | | | | | | |
| T4 | | | | | | | | | |

Figure 11 - The Access Units containing reads of class P mapped on reference sequence no. 2 between position 150,000 and 250,000 are accessed using the values contained in the T1p vector

Reference sequence 1

| A | A | C | T | G | G | A | T | T | C | G | C | C | A |

| A | A | C | T | G | G |

| A | C | T | G | G | A |

| C | T | **C** | G | A | **A** |

| T | **C** | G | A | **A** | T |

Mismatching positions

Class M reads with respect to ref. seq. 1

Reference sequence 2

| A | A | C | T | **C** | G | A | **A** | T | C | G | C | C | A |

"Adapted" reference sequence

| C | T | **C** | G | A | **A** |

| T | **C** | G | A | **A** | T |

Class P reads with respect to ref. seq. 2

**Figure 12 - A modification in the reference sequence can transform M reads in P reads.**

131
Raw sequence data

132

133
Aligned genomic data

134

135
Reads classes

136

Genome Sequencing

Aligner

Reads classificator

Layers encoder

130

137
Syntax elements layers

1316
Compressed data

1312

1311
MIT and AUs

1310

139
AUs with header

138

Storage

Entropy Compressor

MIT encoder

Header and AU encoder

1315

Updated data

1319

Decompression

Analysis/ Update

Genome Sequencing

1313
Decompressed data

1314

1318

1317

**Figure 13 - Overall Architecture.**

24

**Figure 14 – Sequence reads extractor**

**Figure 15 - Genomic encoder**

**Figure 16 – Genomic Decoder**

**EP 3 526 709 B1**

**Patent documents cited in the description**

- US 20150227686 A1